Europäisches Patentamt
European Patent Office
Office européen des brevets

(19)

(11) Publication number: **0 160 565**
**A2**

(12)

# EUROPEAN PATENT APPLICATION

(21) Application number: 85303032.8

(22) Date of filing: 29.04.85

(51) Int. Cl.[4]: C 07 D 501/46

(30) Priority: 30.04.84 GB 8410993

(43) Date of publication of application: 06.11.85 Bulletin 85/45

(84) Designated Contracting States: AT BE CH DE FR GB IT LI NL SE

(71) Applicant: GLAXO GROUP LIMITED
Clarges House 6-12 Clarges Street
London W1Y 8DH(GB)

(72) Inventor: Looker, Brian Edgar
28 Middleton Avenue
Greenford Middlesex(GB)

(74) Representative: Pett, Christopher Phineas et al,
Frank B.Dehn & Co. Imperial House 15-19 Kingsway
London WC2B 6UZ(GB)

(54) **Process for preparing cephalosporin compounds.**

(57) There is described a process for the preparation of compounds of general formula (I)

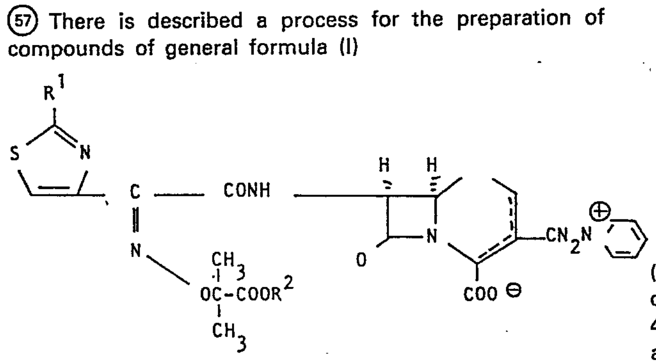

(wherein $R^1$ represents an amino or protected amino group, $R^2$ is hydrogen or a carboxyl blocking group, B is S or S → O and the dotted line indicates that the compound is a ceph-2-em or ceph-3-em compound) or a corresponding compound having a group $-COOR^3$ (wherein $R^3$ is a carboxyl blocking group) at the 4-position and an associated anion $A^{\ominus}$ which comprises reacting a compound of formula (II)

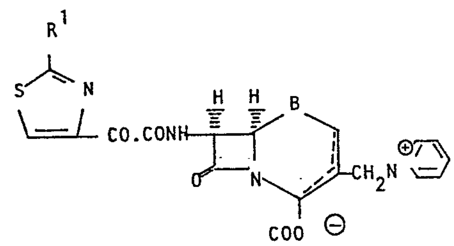

(II)

(wherein $R^1$, B and the dotted line are as defined above) or a corresponding compound having a group $-COOR^3$ at the 4-position (where $R^3$ is as described above) and an associated anion $A^{\ominus}$ with a hydroxylamine derivative of formula (III)

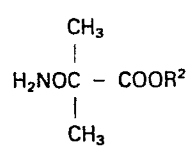

(III)

(wherein $R^2$ is as defined above), or a salt or Zwitterion thereof. The process is of value as a means of preparing the antibiotic ceftazidime. Intermediates of value in the process and of methods of preparing them are also disclosed.

EP 0 160 565 A2

2YX143-404

"Process for preparing cephalosporin compounds"

This invention relates to improvements in or relating to cephalosporins. More particularly it relates to processes for the preparation of the cephalosporin antibiotic ceftazidime.

Ceftazidime ((6R,7R)-7-[(Z)-2-(2-aminothiazol-4-yl)-2-(2-carboxyprop-2-oxyimino)acetamido]-3-(1-pyridiniummethyl)ceph-3-em-4-carboxylate) is a valuable antibiotic having broad spectrum activity, in particular, unusually high activity against gram-negative organisms, including *Pseudomonas* organisms, as described in UK Patent Specification No. 2025398.

Semi-synthetic cephalosporins may conventionally be prepared by the acylation of an appropriate 7-aminocephalosporin with a compound serving to introduce a pre-formed 7-substituent, or by modification at the 3-position, for example the reaction of a 3-acyloxymethyl or 3-halomethyl cephalosporin with a nucleophile, to introduce the desired 3-substituent. These general processes are described in UK Patent Specification No. 2025398 for the preparation of ceftazidime.

We have now devised a process for the preparation of ceftazidime and salts and protected derivatives thereof in which the 2-carboxyprop-2-oxyimino moiety in the 7- side chain of ceftazidime is formed as the last major chemical stage in the synthesis by an oximation reaction.

According to one aspect of the invention, therefore, we provide a process for the preparation of compounds of general formula (I)

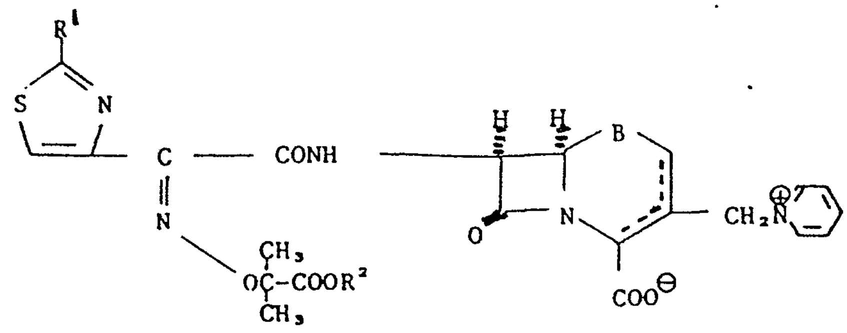

(wherein $R^1$ represents an amino or protected amino group, $R^2$ is hydrogen or a carboxyl blocking group, B is $>$S or $>$S→O and the dotted line indicates that the compound is a ceph-2-em or ceph-3-em compound) or a corresponding compound having a group $-COOR^3$ (where $R^3$ is a carboxyl blocking group) at the 4-position and an associated anion $A^{\ominus}$ which comprises reacting a compound of formula (II)

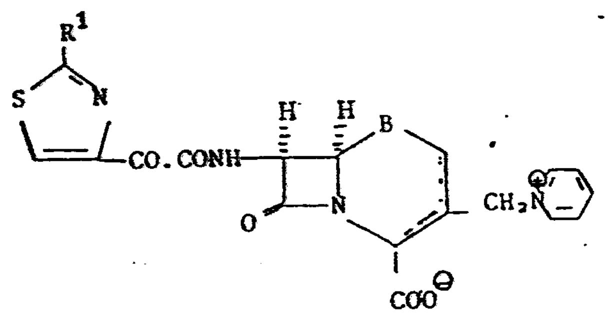

(II)

(wherein $R^1$,B and the dotted line are as defined above) or a corresponding compound having a group $-COOR^3$ at the 4-position (where $R^3$ is as defined above) and an associated anion $A^{\ominus}$ with a hydroxylamine derivative of formula (III)

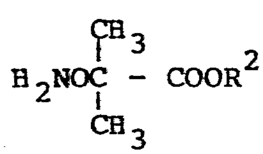

(III)

(wherein $R^2$ is as defined above), or a salt or Zwitterion thereof.

The anion $A^{\ominus}$ which may be associated with a compound of formula (I) or (II) may be derived from an organic or inorganic acid, such as an alkyl-, aryl- or aralkyl- carboxylic or -sulphonic acid or a mineral acid. Examples of such acids include

formic, trifluoroacetic, methanesulphonic, p-toluenesulphonic, hydrochloric, hydrobromic, phosphoric and sulphuric acids.

The reaction may be effected in aqueous or non-aqueous reaction media, conveniently at a temperature in the range -20 to 100°C, e.g. 25-70°C, preferably about 50°C. Solvents which may be employed include water, alcohols e.g. methanol or ethanol; amides e.g. N,N-dimethylformamide, N,N-dimethylacetamide or hexamethylphosphoramide; ethers e.g. cyclic ethers such as tetrahydrofuran or dioxan, and acylic ethers such as dimethoxyethane or diethylether; nitriles e.g. acetonitrile; nitroalkanes e.g. nitromethane; sulphoxides e.g. dimethylsulphoxide; sulphones e.g. sulpholane; hydrocarbons such as halogenated hydrocarbons e.g. methylene chloride; and esters such as ethyl acetate, as well as mixtures of two or more such solvents.

If the oximation is carried out in an anhydrous reaction medium a Lewis acid, such as $BF_3$ conveniently in the form of a solvate e.g. an etherate; magnesium chloride or zinc chloride, is desirably present.

When aqueous conditions are employed the reaction may conveniently be effected at a pH in the range from 2.0 to 9.0, preferably from 3 to 8. The pH may conveniently be maintained in this range by the addition of an appropriate acid or base, for example a mineral acid such as hydrochloric or sulphuric acid or an alkali metal carbonate, or bicarbonate e.g. sodium bicarbonate.

The oxime formation reaction may be followed where necessary and/or desired by conversion of the compound of formula (I) initially obtained into a different compound of formula (I), for example by means of one or more of the following reactions:-

(i) reduction of a compound wherein B is $>S\rightarrow O$ to a compound wherein B is $>S$;

(ii) conversion of a $\Delta^2$- isomer into a $\Delta^3$-isomer;

(iii) removal of any carboxyl-blocking or amino protecting groups; and

(iv) formation of a non-toxic salt or a non-toxic metabolically labile ester.

These reactions may be effected by conventional methods and in any convenient order.

Thus, if desired, a $\Delta^2$-cephalosporin obtained in accordance with the process of the invention may be converted into the corresponding $\Delta^3$-derivative by, for example, treatment of the $\Delta^2$-derivative with a base, such as pyridine or triethylamine.

A ceph-2-em reaction product may also be oxidised to yield the corresponding ceph-3-em 1-oxide, for example by reaction with a peracid, e.g. peracetic or m-chloroperbenzoic acid; the resulting sulphoxide may subsequently be reduced as described hereinafter to yield the corresponding ceph-3-em sulphide.

Where a compound is obtained in which B is $>S\rightarrow O$ this may, if desired, be converted into the corresponding sulphide by, for example, reduction of the corresponding acyloxysulphonium or alkoxysulphonium salt prepared <u>in situ</u> by reaction with e.g. acetyl chloride in the case of an acetoxysulphonium salt, reduction being effected by, for example, sodium dithionite or by iodide ion as in a solution of potassium iodide in a solvent e.g. acetic acid, acetone, tetrahydrofuran, dioxan, dimethylformamide or dimethylacetamide. The reaction may be effected at a temperature of from $-20^{o}$ to $+50^{o}C$.

Carboxyl blocking groups which may be present in the compounds of general formula (I) or any of the intermediates described herein may be any of the conventional carboxyl protecting groups known

in the art, a list of representative protected carboxyl groups being included in British Patent No. 1399086. The carboxyl blocking group may be for example the residue of an ester-forming aliphatic or araliphatic alcohol or an ester-forming phenol, silanol or stannanol preferably containing 1-20 carbon atoms. Thus, carboxyl blocking groups which may conveniently be employed include aryl lower alkyl groups, such as p-methoxybenzyl, p-nitrobenzyl and diphenylmethyl; lower alkyl groups such as t-butyl, lower haloalkyl groups such as 2,2,2-trichloroethyl; and tri(lower alkyl) silyl groups such as trimethylsilyl.

An amino protecting group present in the compounds of formula (I) or intermediates therefor is conveniently a group which may be readily removed at an appropriate stage in the reaction sequence, for example an aralkyl group, such as triphenylmethyl, or an acyl group, such as chloroacetyl, formyl, t-butoxycarbonyl, 2,2,2-trichloroethoxycarbonyl or isoamyloxycarbonyl.

Any carboxyl blocking or amino protecting group present in the compound of formula (I) may be removed, if desired, by any appropriate methods known in the art. Suitable methods for removing carboxyl protecting groups include acid or base hydrolysis and reduction. Thus, for example diphenylmethyl and t-butyl carboxyl blocking groups may conveniently be removed by acid hydrolysis. A p-nitrobenzyl group may conveniently be cleaved by reduction, effected for example by zinc and acetic acid, or sodium dithionite or iodide ion in water, conveniently in the presence of a water-miscible solvent such as acetone, tetrahydrofuran, dioxan, dimethylformamide or dimethylacetamide.

Methods of removing amino protecting groups are also well known in the art. A trityl group may for example be removed using an optionally halogenated carboxylic acid e.g. acetic acid, formic acid, chloroacetic acid or trifluoroacetic acid or using a mineral acid, e.g. hydrochloric acid or mixtures of such

acids, preferably in the presence of a protic solvent such as water. Acid-labile acyl amino-protecting groups, e.g. formyl, t-butoxycarbonyl or isoamyloxycarbonyl may also be removed, where appropriate under the conditions described above for a trityl group. Other acyl amino-protecting groups e.g. 2,2,2-trichloroethoxycarbonyl may be removed, for example, by reduction.

It will be appreciated that the use of amino protecting and carboxyl blocking groups is well known in the art. Relevant examples of such use are given in e.g. Theodora W. Greene "Protective Groups in Organic Synthesis" (Wiley-Interscience, New York 1981), and J.F.W. McOmie, "Protective Groups in Organic Chemistry" (Plenum Press, London 1973).

Salt derivatives of the compounds of formula (I) include inorganic base salts such as alkali metal salts (e.g. sodium and potassium salts) and alkaline earth metal salts (e.g. calcium and magnesium salts); ammonium salts; amino acid salts (e.g. lysine and arginine salts); organic base salts (e.g. procaine, phenylethylbenzylamine, dibenzylethylenediamine, ethanolamine, diethanolamine and N-methyl-glucosamine salts), and acid addition salts, e.g. formed with a mineral acid such as hydrochloric, hydrobromic, sulphuric, nitric or phosphoric acid, or with an organic acid such as formic, acetic, trifluoroacetic, methanesulphonic or p-toluenesulphonic acid. The salts may also be in the form of resinates formed with, for example, a polystyrene resin or crosslinked polystyrene divinylbenzene copolymer resin containing amino or quaternary amino groups or sulphonic acid groups, or with a resin containing carboxyl groups, e.g. a polyacrylic acid resin.

It will be appreciated that when the compound of formula (I) prepared according to the invention is ceftazidime itself, salt and ester derivatives

should be non-toxic and physiologically acceptable, such are described in UK Patent Specification No. 2025398.

Base salts of formula (I) may be formed by reacting an acid of formula (I) with an appropriate base. Thus, for example, sodium or potassium salts may be prepared using the respective 2-ethylhexanoate or hydrogen carbonate salt. Acid addition salts may be prepared by reacting a compound of formula (I) with the appropriate acid.

The reaction product may be separated from the reaction mixture, which may contain, for example, unchanged cephalosporin starting material and other substances, by a variety of processes including solvent extraction, recrystallisation, ionophoresis, column chromatography and use of ion-exchangers (for example by chromatography on ion-exchange resins) or macroreticular resins.

Where a compound of formula (I) is obtained as a mixture of isomers, the syn isomer may be obtained by, for example, conventional methods such as crystallisation or chromatography.

A preferred embodiment of the invention relates to the preparation of ceftazidime, the compound of formula (I) in which $R^1$ is an amino group, $R^2$ is a hydrogen atom, B represents $\rangle$S and the dotted line represents a ceph-3-em compound.

Compounds of formula (II) and their salts are novel compounds. In addition to their utility as intermediates in the process of the present invention the compounds of formula (II) wherein $R^1$ represents an amino group exhibit antibacterial activity against a range of gram-positive and gram-negative organisms, such as Staphylococcus aureus and Escherichia coli. Antibiotic compounds of formula (II) may be used to treat a variety of bacterial infections. They may be formulated for administration in any conventional manner, for

example as described in UK Patent No. 2025398. Accordingly, as a further aspect of the invention we provide compounds of formula (II) as defined above, and salts thereof and processes for their preparation.

A compound of formula (II) may be prepared for example by reacting a compound of formula (IV)

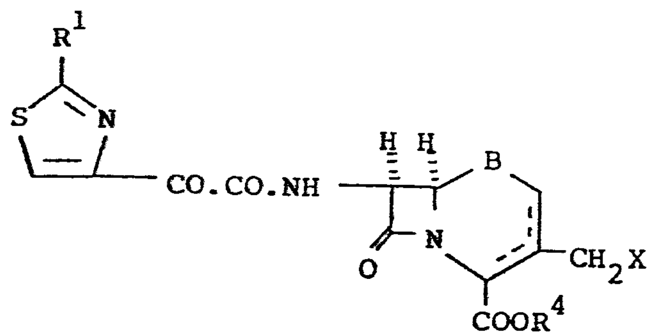

(IV)

wherein $R^1$, B and the dotted line are as hereinbefore defined, $R^4$ represents hydrogen or a carboxyl blocking group and X is a replaceable residue of a nucleophile, e.g. an acetoxy or dichloroacetoxy group or a halogen atom such as chlorine, bromine or iodine) or a salt thereof, with pyridine.

The nucleophilic displacement reaction may be carried out using conventional procedures, for example as described in UK Patent No. 2025398.

Thus, when X represents an acetoxy or substituted acetoxy group the reaction is advantageously effected in an aqueous medium, $R^4$ preferably represents a hydrogen atom and B is preferably $>S$. When X is acetoxy the reaction may be facilitated by the presence in the reaction medium of iodide or thiocyanate ions as described in British Patent Specifications 1,132,621 and 1,171,603.

When a compound of formula (IV) wherein X represents a halogen atom is employed, B may represent $>S\rightarrow O$ and $R^4$ may represent a carboxyl blocking group. The reaction is conveniently effected in a non-aqueous medium which preferably comprises one or more organic solvents, advantageously of a polar nature, such

as ethers, esters, amides or ketones, or pyridine itself may act as the solvent.

A compound of formula (IV) may be prepared by acylating a corresponding 7-amino compound, for example as described in British Patent No. 1575803.

The invention will now be more particularly described in the following Preparation and Examples. All temperatures are in $^{o}$C. Sorbsil U30 is silica gel manufactured by Joseph Crosfield and Son of Warrington, Cheshire, England. Amberlite XAD-2 is a non-functional macroreticular resin manufactured by Rohm and Haas of Philadelphia USA.

## Preparation

t-Butyl 2-methyl-2-phthalimido-N-oxypropionate

N-Hydroxyphthalimide (16.3g) was stirred with t-butyl 2-bromoisobutyrate (27g) and potassium carbonate (11g) in dimethyl sulphoxide (100ml) at 21$^{o}$ for ten days. The mixture was partitioned between water and ethylacetate, further sodium bicarbonate being added to raise the mixture to pH8. The organic layer was washed with water and brine and dried with magnesium sulphate. The solvent was evaporated and the residue was triturated with petroleum ether (bp 40 to 60$^{o}$) to give a solid. This solid was recrystallised from ethanol to give the title compound (14.75g) mp 105-107$^{o}$, λmax(EtOH) 294nm($E^{1\%}_{1cm}$ 60), λinfl 240nm($E^{1\%}_{1cm}$ 297), λinfl 298 nm ($E^{1\%}_{1cm}$ 59).

## Example 1

t-Butyl [6R,7R]-3-Acetoxymethyl-7-(2-triphenylmethylaminothiazol-4-yl)glyoxamidoceph-3-em-4-carboxylate.

2-Triphenylmethylaminothiazol-4-ylglyoxylic acid (1.3g) was stirred in methylene chloride (20ml) at -25$^{o}$ and triethylamine (0.43ml) was added. Pivaloyl

chloride (0.38ml) in methylene chloride (10ml) was added over four minutes so that the temperature remained below $-20^{O}$. The solution was stirred with ice-water cooling for three hours, then cooled to $-25^{O}$ and a solution of t-butyl 7-aminocephalosporanate (1.22g) in methylene chloride (25ml) was added. The resulting solution was allowed to warm to $21^{0}$ and was stirred at this temperature for two hours. The solution was evaporated and the residue was triturated with diethyl ether. The solid obtained and the evaporated mother-liquors were recombined and chromatographed on Sorbsil U30 (50g) in ethyl acetate (10 to 50%) in petroleum ether (bp 40 to $60^{O}$) to give the title compound (1.5g), $[\alpha]_D^{21}$ $+31.9^{O}$( $\underline{C}$ 0.97,$CHCl_3$).

Example 2

[6R,7R]-3-Acetoxymethyl-7-(2-aminothiazol-4-yl)glyoxamido-ceph-3-em-4-carboxylic acid, trifluoroacetate salt.

The product of Example 1 was dissolved in trifluoroacetic acid (6ml) and the solution was stored at $23^{O}$ for 1.5 hours. The solution was then diluted with diisopropyl ether (50ml) and the precipitate was collected by filtration, washed with diisopropyl ether and dried to give the title compound (850mg), λmax(pH6 buffer) 245nm ($E_{1cm}^{1\%}$ 301), 337nm($E_{1cm}^{1\%}$ 73).

Example 3

[6R,7R]-7-(2-Aminothiazol-4-ylglyoxamido)-3-(1-pyridiniummethyl)ceph-3-em-4-carboxylate.

The product of Example 2 was mixed with sodium bicarbonate (230mg) and water (0.7ml) was added. The mixture was heated to $50^{O}$, and to the resulting suspension were added sodium iodide (3g) followed by pyridine (0.28ml) was added, to give a solution. Further sodium bicarbonate was added to adjust the pH of this solution from pH6.5 to 7.0. The mixture

was heated to 80° for one hour, maintaining pH 7.0 by adding more sodium bicarbonate. After cooling, the mixture was diluted with acetone and the precipitate was collected by filtration, washed with acetone and dried to give a solid (690mg). This was dissolved in water and applied to a column of Amberlite XAD-2 resin (100g) in water. After removing inorganic material with water, elution was continued with 25% ethanol in water. Combination and evaporation of the appropriate eluate gave a gum which was triturated with acetone to give the <u>title compound</u> (150mg), λmax(pH6 buffer) 252.5nm($E^{1\%}_{1cm}$ 332),336nm($E^{1\%}_{1cm}$ 71).

<u>Example 4</u>

<u>(6R,7R,)-7-[(Z)-2-(2-Aminothiazol-4-yl)-2-(2-carboxyprop-2-oxyimino)acetamido]-3-(1-pyridiniummethyl)ceph-3-em-4-carboxylate.</u>

The product of the Preparation (3.05g) was dissolved in methylene chloride (30ml) with stirring and a solution of hydrazine hydrate (0.97ml) in methanol (2ml) was added. After 1.5 hours (during which the mixture was occasionally agitated) 5N ammonium hydroxide solution was added to dissolve the solid which had precipitated. The phases were separated and the aqueous phase was extracted twice with methylene chloride. The combined organic layers were washed with water, dried with magnesium sulphate and evaporated to an oil (1.7g). A portion of this oil (44mg) was added to a solution of the product of Example 3 (100mg) in water (4ml) and the solution was adjusted to pH7.0 by addition of very dilute sodium bicarbonate solution. The solution was heated to 48° for a total of 4.5 hours, during which ethanol (1ml) was added. Dilute hydrochloric acid was added to maintain pH7.0. The mixture was evaporated and the residue was triturated with acetone to give a solid (80mg). This solid was dissolved in trifluoroacetic acid (0.8ml) and

after one hour at $21^{\circ}$, the solution was concentrated. The residue was triturated with diethyl ether to give a solid (38mg) which was shown to contain the title compound by high pressure liquid chromatographic assay in comparison with an authentic sample of ceftazidime, whose spectroscopic characteristics resembled those obtained in Example 3 of UK Patent No. 2025398.

CLAIMS:

1. A process for the preparation of compounds of general formula (I)

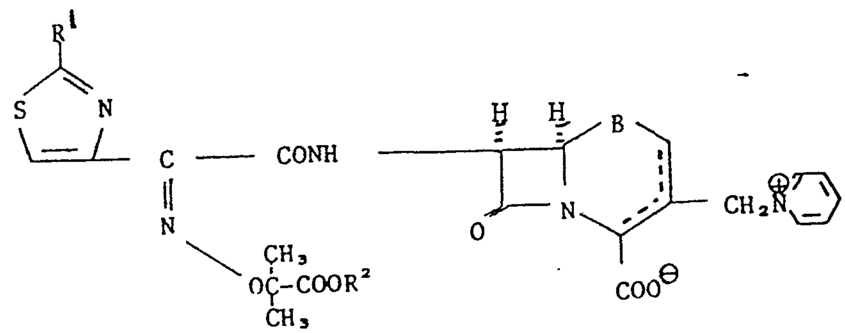

(wherein $R^1$ represents an amino or protected amino group, $R^2$ is hydrogen or a carboxyl blocking group, B is $>S$ or $>S\rightarrow O$ and the dotted line indicates that the compound is a ceph-2-em or ceph-3-em compound) or a corresponding compound having a group $-COOR^3$ (where $R^3$ is a carboxyl blocking group) at the 4-position and an associated anion $A^{\ominus}$ which comprises reacting a compound of formula (II)

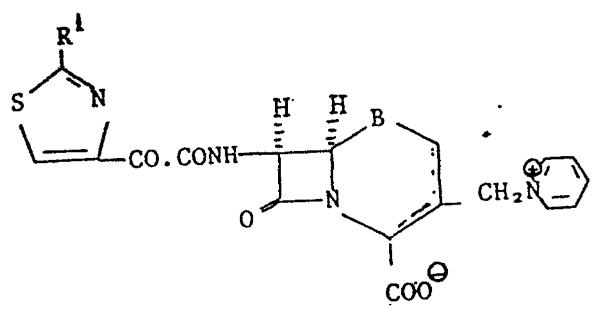

(II)

(wherein $R^1$,B and the dotted line are as defined above) or a corresponding compound having a group $-COOR^3$ at the 4-position (where $R^3$ is as described above) and an associated anion $A^{\ominus}$ with a hydroxylamine derivative of formula (III)

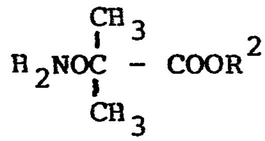

(III)

(wherein $R^2$ is as defined above), or a salt or Zwitterion thereof.

2. A process as claimed in claim 1 wherein reaction is carried out in water, an alcohol, an amide, an ether, a nitrile, a nitroalkane, a sulphoxide, a sulphone, a hydrocarbon or an ester, or a mixture of two or more thereof.

3. A process as claimed in claim 1 or claim 2 wherein if reaction is carried out in an anhydrous reaction medium, a Lewis acid is present.

4. A process as claimed in claim 1 or claim 2 wherein, when aqueous conditions are employed, reaction is carried out at a pH between 3 and 8.

5. A process as claimed in any of claims 1 to 4 wherein the reaction of the compound of formula (II) with the compound of formula (III) is followed, where necessary or desired, by conversion of the compound of formula (I) initially formed into a different compound of formula (I) by means of one or more of the following reactions

(i) reduction of a compound wherein B is $>S\rightarrow O$ to a compound wherein B is $>S$;

(ii) conversion of a $\Delta^2$- isomer into a $\Delta^3$-isomer;

(iii) removal of any carboxyl-blocking or amino protecting groups; and

(iv) formation of a non-toxic salt or a non-toxic metabolically labile ester.

6. A process as claimed in any of claims 1 to 5 wherein, in the compound of formula (I) obtained, $R^1$ represents an amino group, $R^2$ represents a halogen atom or esterifying group, the dotted line represents a ceph-3-em compound and B is $>$S.

7. A compound of formula (II) as defined in claim 1 or a salt thereof.

8. A process for the preparation of a compound of formula (II) or a salt thereof as defined in claim 1 which comprises reacting a compound of formula (IV)

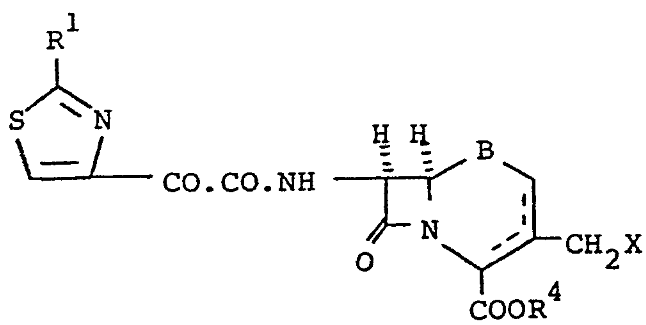 (IV)

wherein $R^1$, B and the dotted line are as hereinbefore defined, $R^4$ represents hydrogen or a carboxyl blocking group and X is a replaceable residue of a nucleophile, e.g. an acetoxy or dichloroacetoxy group or a halogen atom such as chlorine, bromine or iodine) or a salt thereof, with pyridine.

9. [6R,7R]-7-(2-Aminothiazol-4-ylglyoxamido)-3-(1-pyridiniummethyl)ceph-3-em-4-carboxylate.

10. [6R,7R]-3-Acetoxymethyl-7-(2-aminothiazol-4-yl)glyoxamidoceph-3-em-4-carboxylic acid, trifluoroacetate salt.

11. t-Butyl[6R,7R]-3-Acetoxymethyl-7-(2-triphenylmethylaminothiazol-4-yl)glyoxamidoceph-3-em-4-carboxylate.